Europäisches Patentamt

European Patent Office

Office européen des brevets

(19)

(11) Veröffentlichungsnummer : **0 431 465 B1**

(12) **EUROPÄISCHE PATENTSCHRIFT**

(45) Veröffentlichungstag der Patentschrift :
13.04.94 Patentblatt 94/15

(51) Int. Cl.$^5$ : **A61K 33/00,** A61K 31/19,
// (A61K33/00, 31:70, 31:195,
31:19)

(21) Anmeldenummer : 90122823.9

(22) Anmeldetag : 29.11.90

(54) **Dialysier- und Spüllösung zur intraperitonealen Verarbreichung.**

(30) Priorität : 04.12.89 DE 3940052

(43) Veröffentlichungstag der Anmeldung :
12.06.91 Patentblatt 91/24

(45) Bekanntmachung des Hinweises auf die
Patenterteilung :
13.04.94 Patentblatt 94/15

(84) Benannte Vertragsstaaten :
AT BE CH DE DK ES FR GB GR IT LI NL SE

(56) Entgegenhaltungen :
EP-A- 0 054 635

(73) Patentinhaber : NEPHRO-MEDICA
PHARMAZEUTISCHE
VERTRIEBSGESELLSCHAFT MBH
Giessener Strasse 115
D-35440 Linden (DE)

(72) Erfinder : Gretz, Norbert, Dr.
Pfalzstrasse 11
W-6800 Mannheim 51 (DE)

(74) Vertreter : Schubert, Siegmar, Dipl.-Ing. et al
Patentanwälte Dr. V. Schmied-Kowarzik Dr. P.
Weinhold Dipl.-Ing. G. Dannenberg Dr. D.
Gudel Dipl.-Ing. S. Schubert Dr. P. Barz Grosse
Eschenheimer Strasse 39
D-60313 Frankfurt (DE)

## Beschreibung

Die vorliegende Erfindung betrifft die Verwendung von alpha-Ketocarbonsäuren als osmotisch wirksame Substanz bei wäßrigen Dialysier- und Spüllösungen zur intraperitonealen Verabreichung.

Die Therapie von Patienten mit akuter oder chronischer Niereninsuffizienz erfolgt in der Weise, daß die eingeschränkte oder völlig fehlende Funktion der Nieren in der Blutreinigung, das heißt das Herausfiltern harnpflichtiger, vor allem stickstoffhaltiger Substanzen und überschüssigen Wassers aus dem Blut und deren Ausscheidung mit dem Harn und die Regulation des Elektrolyt- und Säuren-Basen-Haushalts, durch alternative technische Verfahren kompensiert werden muß. Hierzu werden weltweit praktisch zwei Verfahren angewendet: die Hämodialyse als extrakorporeales Verfahren und die Peritonealdialyse als intrakorporeales Verfahren.

Bei der Hämodialyse wird mittels eines Kurzschlusses zwischen Arterie und Vene ein Shunt angelegt, um einen ausreichend hohen extrakorporealen Blutfluß zu gewährleisten ( 300 bis 500 ml/min ). Aus diesem Shunt wird mit einer großlumigen Nadel das Blut über ein Schlauchsystem zum Dialysator, der künstlichen Niere, geführt. Über eine semipermeable Membran im Dialysator treten überschüssiges Wasser und harnpflichtige Substanzen aus dem Blut in eine Dialysier- und Spüllösung ( Dialysat ) über.

Bei der Peritonealdialyse dient das Peritoneum als semipermeable Membran, über die die harnpflichtigen Substanzen, Elektrolyte und überschüssiges Blutwasser aus dem Blut in das Dialysat übertreten. Die Dialysierlösung, in der Regel 2 l, wird aus Plastikbehältnissen über ein Schlauchsystem und einen dauerhaft implantierten Katheter in die Peritonealhöhle eingeführt. Nach einer bestimmten Equilibrierungszeit, das heißt nach konzentrationsabhängigem Austausch von Stoffen des Dialysats mit dem Blut, wird dann die "verbrauchte" Dialysierlösung über den Katheter abgelassen und durch frische Dialysierlösung ersetzt. Die Peritonealdialyse, zum Beispiel die kontinuierliche ambulante Peritonealdialyse (CAPD) oder die intermittierende Peritonealdialyse (IPD), gewinnt bei diesen technischen Blutreinigungsverfahren zunehmend an Bedeutung. Während in den letzten Jahren der Großteil der durch die Niereninsuffizienz bedingten Manifestationen bei einer Langzeit-Peritonealdialysebehandlung gut beherrscht werden, gibt es eine Anzahl metabolischer Defekte, die sekundär durch die osmotisch wirkende Substanz des Dialysats hervorgerufen werden.

Eine andere, der Peritonealdialyse in der Technik sehr ähnliche Therapieform, ist die Peritoneallavage, die insbesondere in der Chirurgie bei postoperativen Bauchfellentzündungen Anwendung findet. Das Dialysat fungiert hierbei hauptsächlich als Spüllösung.

Eine zentrale Aufgabe einer Dialysierlösung für niereninsuffiziente Patienten besteht einerseits darin, harnpflichtige Substanzen und überschüssiges Wasser aufzunehmen, auf der anderen Seite in der Zufuhr von Substanzen, die metabolisch bedingt in zu geringer Konzentration im Organismus vorkommen (z.B. Elektrolytbilanz oder Aminosäurenbilanz).

Eine weitere Anforderung an eine Peritonealdialysierlösung besteht darin, für den Patienten einen positiven nutritiven Effekt hinsichtlich der Proteinbiosynthese zu erzielen. Dies ist insbesondere im Hinblick darauf von Bedeutung, als der CAPD-Patient täglich einen Verlust von 5 bis 12 g Protein und einigen Gramm Aminosäuren in das Dialysat hat. Weitere Ansprüche an eine Peritonealdialysierlösung sind eine Zusammensetzung aus möglichst physiologischen, also im Organismus natürlich vorkommenden Komponenten, damit eine möglichst hohe Biokompatibilität erreicht wird und weitere pathophysiologische Veränderungen, wie z.B. Akkumulation unphysiologischer oder nicht metabolisierbarer Substanzen im Organismus vermieden werden (vergl.: Henderson, I.S.: Composition of Peritoneal Dialysis Solutions. Blood Purif., 7, 86-94, 1989).

Der Wasserentzug erfolgt bei der Peritonealdialyse durch osmotisch wirksame Substanzen im Dialysat. Die Anforderungen an solche osmotisch wirksamen Substanzen sind, daß sie keine systemische oder peritoneale Toxizität, keine Hemmung der lokalen Immunabwehrmechanismen, keine Immuntoxizität, eine nach Resorption schnelle Metabolisierung bei möglichst langsamer Resorptionsrate und möglichst physiologische pH- und Osmolaritätsbereiche aufweisen. Weitere Anforderungen sind, daß sie einen adäquaten nutritiven Beitrag leisten und bei geringen Kosten einfach zu produzieren und technisch zu handhaben sind. Bekannte osmotisch wirksame Substanzen, die für Peritonealdialysierlösungen vorgeschlagen wurden, sind z.B. Glukose, Fruktose, Zuckeralkohole und -derivate, Polymere, Dextrane, Glycerin, Gelatin, Albumin oder Aminosäuren, wobei bis heute noch keine ideale osmotisch aktive Substanz gefunden wurde. Die Anforderungen an solche Substanzen und die Nachteile der bisher bekannten osmotisch aktiven Substanzen in Peritonealdialysierlösungen sind in Übersichtsartikeln, wie z.B. in "Hain, H., Kessel, M.: Aspects of New Solutions for Peritoneal Dialysis. Nephrol.Dial.Trans-plant., 2, 67-72, 1987" oder in "Mistry, C.D., Gokal, R.: Alternative Osmotic Agents. Blood Purif., 7, 109-114, 1989" publiziert.

Weitaus am verbreitesten wurde bisher Glukose als osmotisch wirksame Substanz eingesetzt. So enthalten alle kommerziell verfügbaren Peritonealdialysierlösungen Glukose als osmotisch wirksame Substanz. Glukose zeigt jedoch folgende Nachteile : pro Tag absorbieren CAPD-Patienten 150 bis 300 g Glukose vom Dialysat. Diese Menge entspricht ungefähr 2500 bis 5000 kJ (600 bis 1200 kcal) und entspricht damit ungefähr einem Drittel des täglichen Energiebedarfs (Beitrag zur Obesitas der CAPD-Patienten). Die Blutglukosekonzentrationen sind erhöht (Hyperglycämie) mit der Folge einer erhöhten Insulinkonzentration (Hyperinsulinämie) oder bei diabetischen Patienten eines erhöhten Insulinbedarfs. Hyperglycämien führen des weiteren zu Hyperlipidämien, wodurch als Folge atherosklerotische Veränderungen hervorgerufen werden können.

Um diese Nachteile und Probleme, die mit der Verwendung von Glukose als osmotisch wirksame Substanz in Dialysierlösungen verbunden sind, auszuschalten, wurden zahlreiche Untersuchungen hinsichtlich alternativer osmotisch wirksamer Substanzen unternommen (vergl. o.g. Literaturhinweise Hain und Kessel oder Mistry und Gokal).

Wird anstelle von Glukose die als physiologisch zu kennzeichnende Fruktose als osmotisch wirksame Substanz verwendet, treten häufig Irritationen des Peritoneums auf. Werden zwar die bei Glukose auftretenden Probleme der Hyperglycämie durch die Verwendung von Fruktose umgangen, wie das z.B. auch beim Austausch von Glukose durch Fruktose in der Ernährung bei Diabetes genutzt wird, so führen erhöhte Fruktoseplasmakonzentrationen, insbesondere bei erhöhten Blutglukosespiegeln (Diabetes mellitus), über den Polyolweg zu einem Anstieg der Fruktosekonzentration im Linsengewebe des Auges. Im Gegensatz zu Glukose kann aber Fruktose das Linsengewebe nicht mehr durch Permeation verlassen und die Fruktosekonzentration in dem Maß ansteigen, daß osmotisch wirksame Konzentrationen von Fruktose im Linsengewebe auftreten mit der Folge eines Wassereinstroms in das Linsengewebe mit Schwellung und Störung der optischen Eigenschaften bis letztendlich zum Zustandsbild des diabetischen Katarakts (Linsentrübung).

Aus der PCT-PA WO 83/00087 ist eine wäßrige Dialysierlösung bekannt, die sich dadurch auszeichnet, daß sie metabolisierbare Kohlenhydratpolymere als osmotisch wirksame Substanz enthält, die einen durchschnittlichen Polymerisationsgrad von 4 aufweisen, vorzugsweise ist das Kohlenhydratpolymer ein Glukosepolymer mit einem Polymerisationsgrad von 4 bis 10.

Der Abfall in der Osmolarität der Dialysierlösung während der intraperitonealen Verweildauer soll bei Glukosepolymeren als osmotisch wirksame Substanz geringer sein als bei Mono- oder Disacchariden enthaltenden Dialysierlösungen. Da Glukosepolymere unphysiologische Substanzen sind, entsprechen sie nicht den Anforderungen an ideale osmotisch wirksame Substanzen, im besonderen im Hinblick auf die Gefahr von Akkumulationen im Organismus.

Letzteres gilt insbesondere im Hinblick auf negative Langzeitwirkungen unphysiologischer osmotisch aktiver Substanzen für Dialysierlösungen, die hauptsächlich auf einer zu geringen oder zu langsamen Metabolisierung der Substanzen, nach Resorption über das Peritoneum, beruhen.

In der wissenschaftlichen Literatur sind z.B. hyperosmolare Plasmazustände bei der intraperitonealen Verabreichung der Zuckeralkohole Sorbit und Xylit, Kumulation von Glukosepolymeren und Glycerin, allergische Reaktionen bei Gelatine und Toxizität von synthetischen Polymeren beschrieben. Außerdem haben höhermolekulare Substanzen, wie z.B. Dextrane, Hydroxyäthylstärke und Albumin, eine geringere osmotische Aktivität im Vergleich zu kleineren Molekülen, so daß, um einen adäquaten Wasserentzug zu gewährleisten, hohe Konzentrationen eingesetzt werden müssen, welche wiederum die Gefahr von Schockreaktionen erhöhen (z.B. bekannt als Dextranschock).

Als weitere Möglichkeit der Verwendung alternativ osmotisch wirkender Substanzen in Peritonealdialysierlösungen wurde bereits seit längerem vorgeschlagen, Aminosäurenlösungen anstelle von Glukose einzusetzen (in: Gjessing, J.: Addition of Amino Acids to Peritoneal Dialysis Fluids. Lancet, Vol.2, 812, 1968). Eine solche wäßrige Dialysierlösung wird auch in der PCT-Patentanmeldung WO 82/03773 vorgeschlagen, enthaltend ein Gemisch physiologischer Aminosäuren, vorzugsweise essentielle Aminosäuren, Insulin und ein Kohlenhydrat in ausreichender Menge, um für den Patienten eine Assimilation der Aminosäuren zu gewährleisten. Als geeignetes Aminosäurengemisch ist in der PCT-Patentanmeldung das Handelsprodukt Travasol[R] genannt. Vorzugsweise enthalten die Dialysierlösungen gemäß dieser Patentanmeldung die Mengen 1 bis 4 g Aminosäuren/l, 0,5 bis 4 g Glukose/l und Insulin.

Durch solche Dialysierlösungen werden zwar die Probleme der Hyperglycämie vermindert und ausreichende osmotische Effekte erzielt, jedoch ist es mit diesen bekannten aminosäurenhaltigen Dialysierlösungen bisher nicht gelungen, einen ausreichenden nutritiven Effekt bei niereninsuffizienten Patienten zu erreichen, vor allem wegen der noch nicht gelösten optimalen Aminosäurenzusammensetzung bezüglich des Verhältnisses der essentiellen oder nicht-essentiellen Aminosäuren zueinander und der daraus resultierenden Probleme einer mangelnden Proteinbiosynthese, metabolischer Aminosäurenimbalanzen, Anstieg der Harnstoffwerte und Verschlechterung der Acidose (Säure-Basen-Haushalt) (vergl. Lindholm, B., Tranaeus, A., Werynski, A., Österberg, T., Bergström, J.: Amino Acids for Peritoneal Dialysis: Technical and metabolic implications. Perit.Dial.Bulletin, 6, 149-154, 1986 oder Young, G.A., Dibble, J.B., Hobson, S.M., Tomkins, L., Gibson J. Turney, J.H., Brownjohn, A.M.: The Use of an Amino-acid-based CAPD Fluid over 12 Weeks. Nephrol.Dial.Transplant., 4, 285-292, 1989).

Bei der intraperitonealen Verabreichung von Aminosäuren bei niereninsuffizienten Patienten wird der kran-

EP 0 431 465 B1

ke Organismus zusätzlich mit stickstoffhaltigen Substanzen (Aminosäuren) belastet, die wiederum nach Metabolisierung den Großteil der harnpflichtigen Substanzen, die eliminiert werden müssen, darstellen. Die Folge ist die Notwendigkeit der Einhaltung einer strikten Diät.

Ein weiterer bedeutender technischer Nachteil aminosäuren-und kohlenhydrathaltiger (z.B. Glukose oder Fruktose) Dialysierlösungen liegt darin, daß die Aminogruppen der Aminosäuren mit den Keto-, Aldehyd- oder Carbonylgruppen der Kohlenhydrate (reduzierende Zucker, wie z.B. Glukose, Galaktose oder Fruktose) chemisch reagieren. Diese bereits 1912 durch Maillard beschriebene Reaktion wird als nicht-enzymatische Bräunungsreaktion oder Maillard-Reaktion bezeichnet. Es bilden sich sogenannte Melanoide, braune stickstoffhaltige, zum Teil toxische Polymerisationsprodukte mit einem Molekulargewicht bis zu 300.000 Dalton. Durch die Maillard-Reaktion werden vor allem die essentiellen Aminosäuren zerstört. Die Reaktion verläuft in wäßrigen Lösungen abhängig vom pH-Wert (alkalisch: schnell; sauer: verlangsamt) und der Temperatur (bei 0° C sehr langsam, über 90° C sehr schnell). Dies hat zur Folge, daß die unumgänglich notwendige Sterilisation mit Hitze von wäßrigen Aminosäuren-Glukose-Gemischen nicht möglich ist. In der PCT-Patentanmeldung WO 82/03773 wird zur Lösung dieses Problems die Trennung der Aminosäurenlösung und der Glukoselösung in einzelne Behältnisse und Sterilisation in diesen getrennten Behältnissen vorgeschlagen. Das Mischen der beiden getrennten Lösungen über einen die beiden Lösungen verbindenden Konnektor erfolgt unmittelbar vor der intraperitonealen Verabreichung. Daraus ergeben sich Nachteile in der technischen Handhabung und Herstellung solcher Dialysierlösungen. Der damit, im Vergleich zu Fertigdialysierlösungen in einem Behältnis, verbundene höhere technische Aufwand ergibt auch höhere Herstellkosten und somit höhere Preise.

Daher besteht nach wie vor ein erheblicher Bedarf an osmotisch wirksamen Dialysier- und Spüllösungen, die über längere Zeit an niereninsuffiziente Patienten, die mit dem Verfahren der Peritonealdialyse behandelt werden, verabreicht werden können, ohne daß auf die osmotische Wirkung zurückzuführende Komplikationen auftreten, die eine Korrektur der Elektrolytbilanz, eine Korrektur des Säure-Basen-Haushalts, einen ausreichend hohen Entzug von Wasser und harnpflichtigen Substanzen und einen wirksamen nutritiven Effekt bei den Patienten gewährleisten und die desweiteren technisch einfach zu produzieren und technisch einfach in der Handhabung der Verabreichung sind.

Die der Erfindung zugrundeliegende Aufgabe ist es, osmotisch wirksame Dialysier- und Spüllösungen zur Verfügung zu stellen, die über längere Zeit an niereninsuffiziente Patienten intraperitoneal verabreicht werden können, ohne daß auf die osmotische Wirkung zurückzuführende Komplikationen auftreten, wobei die bei bisher bekannten Dialysier- und Spüllösungen auftretenden osmotischen, metabolischen oder peritoneal dialytischen Komplikationen vermieden werden und sowohl ein ausreichender Entzug von Wasser und harnpflichtigen Substanzen, eine Korrektur der Elektrolytbilanz, eine Korrektur des Säure-Basen-Haushalts und ein wirksamer nutritiver Beitrag für den Patienten gewährleistet werden, als auch eine einfache technische Produktion und Handhabung bei der Verabreichung sicherstellen.

Diese Aufgabe wird durch die erfindungsgemäße Dialysier- und Spüllösung zur intraperitonealen Verabreichung, enthaltend Elektrolyte und weitere osmotisch wirksame Substanzen und gegebenenfalls weitere Zusätze, dadurch gelöst, daß in einem Gemisch folgender Bestandteile aus alpha-Ketosäuren in einer (Gesamt-)Konzentration von 1 bis 70 g/l die Bestandteile in folgenden relativen Mengen enthalten sind:

| | | |
|---|---|---|
| alpha-Ketoglutarat | 0 bis 45 | Gew.-Teile |
| Pyruvat (alpha-Ketopropionat) | 0 bis 45 | Gew.-Teile |
| alpha-Ketosuccinat | 0 bis 45 | Gew.-Teile |
| 3-Methyl-alpha-Ketovalerianat | 6 bis 9 | Gew.-Teile |
| 4-Methyl-alpha-Ketovalerianat | 9 bis 13,5 | Gew.-Teile |
| 3-Phenylpyruvat | 0 bis 9,5 | Gew.-Teile |
| 3-Methyl-alpha-Ketobutyrat | 13,5 bis 20,5 | Gew.-Teile |
| alpha-Ketoadipat | 0 bis 10 | Gew.-Teile |
| alpha-Ketobutyrat | 0 bis 10 | Gew.-Teile |

und worin das Verhältnis der alpha-Ketosäuren 3-Methyl-alpha-Ketobutyrat: 4-Methyl-alpha-Ketovalerianat:

4

3-Methyl-alpha-Ketovalerianat vorzugsweise 2,25 : 1,5 : 1 beträgt.

Bei Verwendung eines Gemischs von alpha-Ketosäuren als osmotisch wirksame Substanzen in Dialysier- und Spüllosungen in der Konzentration von 1 bis 70 g/l, sind die folgenden alpha-Ketosäuren in den folgenden relativen Mengen enthalten:

```
alpha-Ketoglutarat                     0 bis 45    Gew.-Teile
Pyruvat (alpha-Ketopropionat)          0 bis 45    Gew.-Teile
alpha-Ketosuccinat                     0 bis 45    Gew.-Teile
3-Methyl-alpha-Ketovalerianat          6 bis  9    Gew.-Teile
4-Methyl-alpha-Ketovalerianat          9 bis 13,5  Gew.-Teile
3-Phenylpyruvat                        0 bis  9,5  Gew.-Teile
3-Methyl-alpha-Ketobutyrat          13,5 bis 20,5  Gew.-Teile
alpha-Ketoadipat                       0 bis 10    Gew.-Teile
alpha-Ketobutyrat                      0 bis 10    Gew.-Teile
```

Bei den erfindungsgemäß eingesetzten alpha-Ketosäuren-Gemischen ist es wesentlich, daß, entsprechend dem Muskelaminosäurenmuster niereninsuffizienter Patienten bestimmte Verhältnisse von 3-Methyl-alpha-Ketobutyrat : 4-Methyl-alpha-keto-valerianat : 3-Methyl-alpha-Ketovalerianat eingehalten werden. So sollte dieses Verhältnis derart sein, daß die Konzentration von 3-Methyl-alpha-Ketobutyrat größer als 4-Methyl-alpha-ketovalerianat größer als 3-Methyl-alpha-Ketovalerianat ist. Vorzugsweise sollte das Verhältnis der alpha-Ketosäuren 3-Methyl-alpha-Ketobutyrat : 4-Methyl-alpha-keto-valerianat : 3-Methyl-alpha-Ketovalerianat 2,25 : 1,5 : 1 betragen.

Bei der Anwendung von erfindungsgemäßen alpha-ketosäurehaltigen Dialysier- und Spüllösungen ergeben sich folgende Vorteile:

Alpha-Ketosäuren (= alpha-Ketocarbonsäuren) sind die Ketoanalogen essentieller und nicht essentieller Aminosäuren, das heißt, daß die Aminogruppen der Aminosäuren durch Transaminierungsreaktionen durch eine Ketogruppe substituiert sind. Diese Reaktionen finden physiologisch im Organismus statt. Die alpha-Ketosäuren sind somit vollkommen physiologische Substanzen, deren biochemische Stoffwechselwege hinreichend bekannt sind. In der Tabelle 1 sind die bei der erfindungsgemäßen Verwendung zur Anwendung, als einzelne oder in Form von Gemischen, kommenden osmotisch wirksamen alpha-Ketosäuren als Ketoanalogen ihrer korrespondierenden Aminosäuren zugeordnet.

```
Tab.: 1. Ketoanaloge (alpha-Ketosäuren) der Aminosäuren
```

| Ketoanalog | Aminosäure |
|---|---|
| alpha-Ketoglutarat | Glutamat |
| Pyruvat (= alpha-Ketopropionat) | Alanin |
| alpha-Ketosuccinat | Arginin |
| 3-Methyl-alpha-Ketovalerianat | Isoleucin |
| 4-Methyl-alpha-Ketovalerianat | Leucin |
| 3-Phenylpyruvat | Phenylalanin |
| 3-Methyl-alpha-Ketobutyrat | Valin |
| alpha-Ketoadipat | Lysin |
| alpha-Ketobutyrat | Threonin |

Durch endogene Aminierung werden aus Ketoanalogen, als Vorstufen der Aminosäuren, die entsprechenden Aminosäuren je nach Bedarf gebildet. Überraschenderweise wurde auch festgestellt, daß endogen essentielle Aminosäuren aus ihren analogen alpha-Ketosäuren, wenn diese anstelle der essentiellen Aminosäuren verabreicht werden, gebildet werden (vergl.: Walser, McK., Lund, P., Rudermann, N.B., Coulter, A.W.: Synthesis of Essential Amino Acids from their Alpha-Ketoanalogues by Perfused Rat Liver and Muscle. J.Clin.Invest., 52, 2865-2877, 1973).

Dieser seit Jahren bekannte Effekt wird therapeutisch mit Erfolg insbesondere bei niereninsuffizienten Patienten genutzt, denen oral alpha-Ketosäuren verabreicht werden, die endogen in Aminosäuren umgewandelt werden, die für die Proteinbiosynthese genutzt werden, dadurch endogen Stickstoff binden, wodurch die Belastung des niereninsuffizienten Organismus mit harnpflichtigen, vor allem stickstoffhaltigen Substanzen vermindert wird (vergl. z.B.: Heidland, A.: Keto-Analoge zur Urämiebehandlung. Praxis-Kurier, 41, 17, 1975 oder: Walser, McK.: Ketoacids in the Treatment of Uremia. Clin. Nephrology, 3, 180-186, 1975 oder: Kopple, J.D, Swendseid, M.E.: Amino Acid and Keto Acid Diets for Therapy in Renal Failure. Nephron, 18, 1-12, 1977).

Derselbe positive Effekt kann auch bei der peritonealen Verabfolgung von alpha-Ketosäuren erzielt werden, da aufgrund des annähernd gleichen Molekulargewichts der Ketoanalogen und ihrer korrespondierenden Aminosäuren, eine ungefähr gleichlaufende intraperitoneale Absorption in das Blut, wie sie von den Aminosäuren her bekannt ist, bei den alpha-Ketosäuren zu erwarten ist. Von besonderer Bedeutung ist hierbei, daß bei niereninsuffizienten CAPD-Patienten speziell die essentiellen verzweigtkettigen Aminosäuren Valin, Leucin und Isoleucin, aber auch Lysin, Tyrosin (Synthese aus Phenylalanin), Threonin und die nicht-essentiellen Aminosäuren Serin, Glycin und Alanin (Synthese aus Pyruvat), sowie Ornithin und Glutamin (beide synthetisiert aus Glutamat) im Verhältnis zu den übrigen Aminosäuren in zu geringer Konzentration vorhanden sind, das heißt, daß Aminosäurenimbalenzen vorhanden sind, die eine Proteinbiosynthese inhibieren.

Durch die Dialysierlösung mit einem Gemisch der in Tabelle 1 aufgeführten alpha-Ketosäuren und dem in Beispiel 1 aufgezeigten Mengenverhältnissen ist es somit möglich, alle o.g. Aminosäurendefizite auszugleichen und dadurch einen wirksamen nutritiven Beitrag für CAPD-Patienten, ohne zusätzliche stickstoffbelastung, zu gewährleisten.

Aminosäurendefizite werden durch die Verwendung der erfindungsgemäßen Dialysierlösung nicht nur durch die Synthese der Aminosäuren aus den analogen alpha-Ketosäuren ausgeglichen, sondern darüberhinaus erfolgt ein verminderter Abbau vorhandener Aminosäuren durch eine genügend hohe Konzentration der analogen alpha-Ketosäuren. Der Grund hierfür liegt darin, daß der Abbau von Aminosäuren physiologischerweise zunächst in einer Transaminierungsreaktion zu den analogen alpha-Ketosäuren erfolgt, die bei entsprechend hoher Konzentration, über eine in der Biochemie üblichen Produkthemmung, den weiteren Abbau von Aminosäuren inhibieren.

Aufgrund des annähernd gleichen Molekulargewichts haben alpha-Ketosäuren auch eine nahezu identische osmotische Wirksamkeit wie die analogen Aminosäuren, so daß eine ausreichende osmotische Aktivität gewährleistet wird, um einen genügenden Entzug von Wasser und harnpflichtigen Substanzen zu erzielen.

Neben des bereits aufgeführten Vorteils der vermiedenen zusätzlichen Stickstoffbelastung bei der erfindungsgemäßen Verwendung von alpha-Ketosäuren im Vergleich zu Aminosäuren als osmotisch wirksame Substanz in Dialysierlösungen, ergeben sich weitere Vorteile in der Weise, daß durch alpha-Ketosäuren, insbesondere alpha-Ketoglutarat, Pyruvat und alpha-Ketosuccinat, keine Aminosäurenimbalanzen induziert werden. Dies ergibt sich aus der Stoffwechselstellung dieser Substanzen. Im Citratzyklus ( = Krebszyklus nach H. A. Krebs, Oxford, Nobelpreis 1954), in den der Kohlenhydrat-, Eiweiß- bzw. Aminosäuren- und Fettstoffwechsel in Form des Pyruvats oder der aktivierten Essigsäure einmünden, nehmen diese alpha-Ketosäuren eine dominierende Schlüsselstellung ein, indem der intermediäre Stoffwechsel dieser Nährstoffe somit über diese alpha-Ketosäuren als Zwischenprodukt abläuft. Durch das beim Abbau entstehende Bicarbonat wird die renale Acidose günstig beeinflußt. Darüberhinaus erfolgt auch der Abbau aller übrigen in Tabelle 1 aufgeführten alpha-Ketosäuren zu Pyruvat oder zur aktivierten Essigsäure. Aufgrund der dadurch gegebenen vielfältigen Stoffwechselmöglichkeiten erfolgt eine Aminierung somit nur bei entsprechendem Bedarf.

Durch diese physiologische Stellung dieser alpha-Ketosäuren im Intermediärstoffwechsel ist die pharmakologisch toxikologische Unbedenklichkeit gewährleistet. Die Nachteile von Hyperglycämien, Akkumulationen oder verzögerten Metabolisierungen, die bei der Verwendung bisher bekannter osmotisch wirksamer Substanzen in Dialysierlösungen auftraten, werden durch die Verwendung von alpha-Ketosäuren als osmotisch wirksame Substanzen in Dialysierlösungen vermieden.

Vorteilhaft sind die alpha-ketosäurenhaltigen Dialysier- und Spüllösungen im Vergleich zu aminosäurenhaltigen Dialysierlösungen, wenn üblicherweise ein Kohlenhydratzusatz in solchen Dialysierlösungen erwünscht wird, da alpha-Ketosäuren aufgrund einer fehlenden Aminogruppe, im Gegensatz zu Aminosäuren, mit Kohlenhydraten keine nicht-enzymatische Bräunungsreaktion (Maillard-Reaktion) eingehen. Daher können alpha-ketosäurenhaltige Dialysierlösungen mit einem Kohlenhydratanteil einfach hergestellt werden. Das Ketosäurengemisch kann problemlos zusammen mit dem Kohlenhydratgemisch in einem einzigen Behältnis hergestellt, hitzesterilisiert (autoklaviert) und gelagert werden. Die Bildung toxischer Melanoide wird vermieden. Desweiteren ist die technische Handhabung bei der Verabreichung der Dialysierlösung einfach und damit sicher in der Anwendung, da im Gegensatz zu aminosäurenmit kohlenhydrathaltigen Dialysierlösungen, ein unmittelbar vor der Verabreichung zu erfolgendes Mischen aus zwei Kompartimenten, entfällt.

Somit ergeben sich bei den erfindungsgemäßen Dialysier-und Spüllösungen mit alpha-Ketosäuren als osmotisch wirksame Substanz anstatt der bisherigen osmotisch aktiven Bestandteile alle die diesbezüglich für die bisherigen Bestandteile bekannten positiven Eigenschaften ohne deren Nachteile. Die neue Dialysier- und Spüllösung ist hervorragend für die intraperitoneale Verabfolgung geeignet.

Die neue Dialysierlösung umfaßt dieselben Komponenten wie bereits bekannte peritoneale Dialysier- und Spüllösungen, jedoch mit der Ausnahme, daß sie osmotisch aktive Substanz, wie z.B. Glukose oder Aminosäuren teilweise bis vollständig durch alpha-Ketosäuren ersetzt ist.

Die Herstellung der neuen Dialysier- und Spüllösungen kann nach den bekannten Verfahren zur Herstellung von glukosehaltigen Dialysierlösungen erfolgen.

Die zur Anwendung kommenden alpha-Ketosäurenkonzentrationen sind abhängig von dem gemischten osmolaren Druck und liegen im Bereich von 1 bis 70 g/l für Peritonealdialysierlösungen. Vorteilhafterweise liegen sie im Bereich von 5 bis 50 g/l, insbesondere 7,5 bis 20 g/l.

Der gesamte osmotische Druck der erfindungsgemäßen Dialysierflüssigkeit beträgt vorteilhafterweise 300 bis 700 mosm/l, insbesondere 320 bis 550 mosm/l.

Der pH-Wert der erfindungsgemäßen Lösung liegt im Bereich von 5,0 bis 8,4.

Alpha-Ketosäuren sind leicht erhältliche Handelsprodukte. Im Chemikalienhandel sind die genannten alpha-Ketosäuren mit einem Reinheitsgrad von mehr als 99% erhältlich. Die Gewinnung erfolgt technisch oder mikrobiologisch.

Wenn die erfindungsgemäße Dialysierlösung neben alpha-Ketosäuren auch Glukose als weitere fakultative, osmotisch wirksame Substanz enthält, so beträgt das Mengenverhältnis vorteilhafterweise 1 : 3 bis 3 : 1. Die Dialysierlösung kann aber gewünschtenfalls auch nicht nur Glukose, sondern auch z.B. Fruktose oder andere Zucker, Zuckeralkohole, Glycerin, Zuckerpolymere, Gelatine, Kohlenhydratpolymere, Hydroxyethylstärke, Dextrane, Aminosäuren und/oder Peptide als weitere, fakultative osmotisch wirksame Substanz(en) enthalten.

Die Elektrolytsalze können in bekannter Weise in Form des Acetats, Lactats, Carbonats, Bicarbonats, Sulfats, Hydroxids und/oder Chlorids vorliegen.

Die Elektrolytzusätze in der erfindungsgemäßen Dialysierlösung liegen vorteilhafterweise in einer Konzentration von 125 bis 152 mmol/l Na+, 0 bis 8 mmol/l K$^+$, 0 bis 3 mmol/l Ca++, 0 bis 2,5 mmol/l Mg$^{++}$, 10 bis 80 mmol/l Ionen ausgewählt aus der Gruppe Lactat, Acetat, Bicarbonat und dem entsprechenden Rest an Chlorid.

Die alpha-Ketosäuren können auch in Form ihrer Natrium-, Kalium-, Magnesium- und/oder Calciumsalze Lösungsbestandteile sein . Durch solche alpha-Ketosäurensalze vermindert sich in der fertigen Dialysierlösung der notwendige Anteil an Chloridionen (korrespondierend zu den Kationen-Elektrolytzusätzen). Dies ist besonders vorteilhaft in Bezug darauf, daß verschiedene Publikationen nicht das Natrium per se, sondern das Chloridsalz des Natriums für die Begünstigung des Bluthochdrucks verantwortlich machen. Darüberhinaus sind Nierenerkrankungen häufig mit einer Hyperchloridämie assoziiert.

Das Vorhandensein der beschriebenen Elektrolytsalze der alpha-Ketosäuren, aus denen ein geringerer Chloridionenbedarf in der fertigen Dialysierlösung resultiert, vermindert also die Bluthochdruckgefahr bei entsprechend gefährdeten Patienten. Im Gegensatz zu herkömmlichen Dialysier- und Spüllösungen, mit einem Chloridanteil von 98 bis 107 mmol/l, ist es durch die neue Dialysier- und Spüllösung möglich, nicht nur die bisher eingesetzten Chloridkonzentrationen vorzusehen , sondern auch alle beliebigen niedrigeren Chloridkonzentrationen, bis hin zu chloridfreien Dialysier- und Spüllösungen.

Zusätzlich können die neuen Dialysierlösungen andere Carbonsäuren oder deren Salze, vorzugsweise ausgewählt aus der Gruppe Succinat, Fumarat, Malat, Citrat oder Isocitrat, in einer Konzentration von 0,5 bis 20 g/l enthalten.

Die neue Dialysier- und Spüllösung kann auch zusätzlich alpha-Hydoxycarbonsäuren oder deren Natrium-, Kalium-, Magnesium- oder Calciumsalze enthalten, vorzugsweise ausgewählt aus der Gruppe Hydroxyphenylpyruvat, insbesondere DL-2-Hydroxy-4-methyl-thiobutyrat enthält.

Die erfindungsgemäße Dialysierlösung kann gewünschtenfalls auch übliche Zusatzstoffe und Hilfsstoffe in pharmakologisch wirksamen Mengen enthalten. Beispiele üblicher Zusatzstoffe und Hilfsstoffe sind Hormone, wie Insulin oder Erythropoetin, Vitamine, Antioxidantien oder Konservierungsstoffe.

Die nachfolgenden Beispiele dienen der weiteren Erläuterung der vorliegenden Erfindung:

Beispiel 1:

In einem Liter Wasser von Injektionsqualität wird eine erfindungsgemäße Dialysier- und Spüllösung hergestellt. Diese Lösung enthält in einem 1 Wasser folgende Mengen an osmotisch wirksamen Substanzen aus alpha-Ketosäuren:

| | |
|---|---|
| alpha-Ketoglutarsäure | 1,5 g/l |
| Pyruvat (alpha-Ketopropionsäure) | 2,0 g/l |
| alpha-Ketosuccinat | 0,9 g/l |
| 3-Methyl-alpha-Ketovaleriansäure | 0,8 g/l |
| 4-Methyl-alpha-Ketovaleriansäure | 1,2 g/l |
| 3-Phenylpyruvat | 0,8 g/l |
| 3-Methyl-alpha-Ketobuttersäure | 1,75 g/l |
| alpha-Ketoadipinsäure | 0,9 g/l |
| alpha-Ketobuttersäure | 0,9 g/l |
| Hydroxy-Phenylpyruvat | 1,0 g/l |

Elektrolytsalze werden in Form des Lactats und Chlorids zugesetzt.

Zusammensetzung:

| | |
|---|---|
| $Na^+$ | 134,0 mmol/l |
| $Ca^{++}$ | 1,75 mmol/l |
| $Mg^{++}$ | 0,75 mmol/l |
| $Cl^-$ | entsprechend (max. 91 mmol/l) |
| $Lactat^-$ | 48,0 mmol/l |
| $alpha-Ketoglutarat^{--}$ | 10,3 mmol/l |
| $Pyruvat^-$ (alpha-Ketopropionat$^-$) | 23,0 mmol/l |
| $alpha-Ketosuccinat^-$ | 6,8 mmol/l |
| $3-Methyl-alpha-Ketovalerianat^-$ | 6,1 mmol/l |
| $4-Methyl-alpha-Ketovalerianat^-$ | 9,1 mmol/l |
| $3-Phenylpyruvat^-$ | 4,8 mmol/l |
| $3-Methyl-alpha-Ketobutyrat^-$ | 15,0 mmol/l |
| $alpha-Ketoadipinat^-$ | 5,6 mmol/l |
| $alpha-Ketobutyrat^-$ | 8,8 mmol/l |
| $Hydroxy-Phenylpyruvat^-$ | 5,6 mmol/l |

Die theoretische Osmolarität dieser Lösung beträgt 377 mosm/l. Der saure pH-Wert dieser Lösung kann gewünschtenfalls durch teilweise Substitution von NaCl durch NaOH oder durch Hinzufügen von HCl exakt eingestellt werden, z.B. auf pH 5,7. Dadurch kann eine entsprechende geringfügige Änderung der Chloridionenkonzentration und Osmolarität erfolgen. Die so hergestellte Lösung wird nach den für die Produktion von Dialysier- und Spüllösungen bekannten Verfahren steril filtriert in Plastikbeutel abgefüllt und hitzesterilisiert.

Beispiel 2:

Beispiel 1 wurde wiederholt, jedoch mit der Ausnahme, daß als osmotisch wirksame Substanzen folgende alpha-Ketocarbonsäuren als Mononatriumsalz eingesetzt wurden:

```
3-Methyl-alpha-Ketovalerianat⁻      6,1   mmol/l
4-Methyl-alpha-Ketovalerianat⁻      9,1   mmol/l
3-Methyl-alpha-Ketobutyrat⁻        15,0   mmol/l
```

Als weitere osmotisch wirksame Substanz wurde Glukose in einer Konzentration von 15 g/l in Form von Glukosemonohydrat (16,5 g/l) eingesetzt. Zusätzlich wurde Kalium in Form von Kaliumchlorid in einer Konzentration von 2,0 mmol/l eingesetzt.

Zusammensetzung:

```
Na⁺                              134,0   mmol/l
Ca⁺⁺                               1,75  mmol/l
Mg⁺⁺                               0,75  mmol/l
K⁺                                 2,0   mmol/l
Cl⁻                               75,8   mmol/l
Laktat⁻                          35,0   mmol/l
3-Methyl-alpha-Ketovalerianat⁻    6,1   mmol/l
4-Methyl-alpha-Ketovalerianat⁻    9,1   mmol/l
3-Methyl-alpha-Ketobutyrat⁻      15,0   mmol/l
Glukose                          15,0   g/l
```

Die theoretische Osmolarität dieser Lösung beträgt 463 mosm/l.

Beispiel 3:

Beispiel 1 wurde wiederholt, jedoch mit der Ausnahme, daß als weitere osmotisch wirksame Substanz Glukose in einer Konzentration von 15 g/l in Form von Glukosemonohydrat (16,5 g/l) eingesetzt wurde.

Zusammensetzung

| | | |
|---|---|---|
| $Na^+$ | 134,0 | mmol/l |
| $Ca^{++}$ | 1,75 | mmol/l |
| $Mg^{++}$ | 0,75 | mmol/l |
| $Cl^-$ | entsprechend (max. 91 | mmol/l) |
| Laktat$^-$ | 48,0 | mmol/l |
| alpha-Ketoglutarat$^{--}$ | 10,3 | mmol/l |
| Pyruvat$^-$ (alpha-Ketopropionat$^-$) | 23,0 | mmol/l |
| alpha-Ketosuccinat$^-$ | 6,8 | mmol/l |
| 3-Methyl-alpha-Ketovalerianat$^-$ | 6,1 | mmol/l |
| 4-Methyl-alpha-Ketovalerianat$^-$ | 9,1 | mmol/l |
| 3-Phenylpyruvat$^-$ | 4,8 | mmol/l |
| 3-Methyl-alpha-Ketobutyrat$^-$ | 15,0 | mmol/l |
| alpha-Ketoadipinat$^-$ | 5,6 | mmol/l |
| alpha-Ketobutterat$^-$ | 8,8 | mmol/l |
| Hydroxy-Phenylpyruvat$^-$ | 5,6 | mmol/l |
| Glukose | 15,0 | g/l |

Die theoretische Osmolarität dieser Lösung beträgt 460 mosm/l.

**Patentansprüche**

1. Wäßrige Dialysier- und Spüllösung zur intraperitonealen Verabreichung, enthaltend Elektrolyte und weitere osmotisch wirksame Substanzen und gegebenenfalls weitere Zusätze, wobei der gesamte osmotische Druck im Bereich von 300 bis 700 mosm/l liegt,
   **dadurch gekennzeichnet**,
   daß in einem Gemisch folgender Bestandteile aus alpha-Ketosäuren in einer (Gesamt-)Konzentration von 1 bis 70 g/l die Bestandteile in folgenden relativen Mengen enthalten sind:

| | | |
|---|---|---|
| alpha-Ketoglutarat | 0 bis 45 | Gew.-Teile |
| Pyruvat (alpha-Ketopropionat) | 0 bis 45 | Gew.-Teile |
| alpha-Ketosuccinat | 0 bis 45 | Gew.-Teile |
| 3-Methyl-alpha-Ketovalerianat | 6 bis 9 | Gew.-Teile |
| 4-Methyl-alpha-Ketovalerianat | 9 bis 13,5 | Gew.-Teile |
| 3-Phenylpyruvat | 0 bis 9,5 | Gew.-Teile |
| 3-Methyl-alpha-Ketobutyrat | 13,5 bis 20,5 | Gew.-Teile |
| alpha-Ketoadipat | 0 bis 10 | Gew.-Teile |
| alpha-Ketobutyrat | 0 bis 10 | Gew.-Teile |

   und worin das Verhältnis der alpha-Ketosäuren 3-Methyl-alpha-Ketobutyrat: 4-Methyl-alpha-Ketovalerianat: 3-Methyl-alpha-Ketovalerianat vorzugsweise 2,25 : 1,5 : 1 beträgt.

2. Lösung nach Patentanspruch 1, **dadurch gekennzeichnet**, daß die alpha-Ketocarbonsäuren als osmotisch wirksame Substanz in Form der Calcium-, Kalium-, Magnesium- oder Natriumsalze dieser Ketocarbonsäuren, vorzugsweise Natriumsalze, eingesetzt werden.

3. Lösung nach Patentanspruch 1 oder 2, **dadurch gekennzeichnet,** daß der osmotische Druck vorzugsweise im Bereich von 320 bis 550 mosm/l liegt.

4. Lösung nach einem der Patentansprüche 1 bis 3, **dadurch gekennzeichnet,** daß sie die alpha-Ketocarbonsäuren in einer Menge von 5 bis 50 g/l, vorzugsweise 7,5 bis 20 g/l enthält.

5. Lösung nach einem der Patentansprüche 1 bis 4, **dadurch gekennzeichnet,** daß der pH-Wert 5,0 bis 8,4 beträgt.

6. Lösung nach einem der Patentansprüche 1 bis 5, **dadurch gekennzeichnet,** daß sie die Elektrolyte in einer Konzentration von 125 bis 152 mmol/l Na$^+$, 0 bis 8 mmol/l K$^+$, 0 bis 3 mmol/l Ca$^{++}$, 0 bis 2,5 mmol/l Mg$^{++}$, 10 bis 80 mmol/l Ionen ausgewählt aus der Gruppe Lactat, Acetat, Bicarbonat und den entsprechenden Rest an Chlorid enthält.

7. Lösung nach einem der Patentansprüche 1 bis 6, **dadurch gekennzeichnet,** daß sie zusätzlich ein Carbonsäuresalz, ausgewählt aus der Gruppe Succinat, Fumarat, Malat, Citrat oder Isocitrat, in einer Konzentration von 0,5 bis 20 g/l enthält.

8. Lösung nach einem der Patentansprüche 1 bis 7, **dadurch gekennzeichnet,** daß sie zusätzlich ein alpha-Hydroxycarbonsäuresalz, vorzugsweise ausgewählt aus der Gruppe Hydroxyphenylpyruvat, insbesondere DL-2-Hydroxy-4-methyl-thiobutyrat enthält.

9. Lösung nach einem der Patentansprüche 1 bis 8, **dadurch gekennzeichnet,** daß sie zusätzlich 0,2 bis 10 Gew.-% Aminosäuren enthält.

10. Lösung nach einem der Patentansprüche 1 bis 9, **dadurch gekennzeichnet,** daß sie zusätzlich 0,2 bis 10 Gew.-% wasserlösliche Kohlenhydrate als weitere osmotisch wirksame Substanzen, vorzugsweise Glukose, Fruktose, Galaktose, Hydroxyethylstärke, Dextrane oder Glukosepolymere enthält.

11. Lösung nach einem der Patentansprüche 1 bis 10, **dadurch gekennzeichnet,** daß sie zusätzlich 10 bis 500 mg/l Vitamine, den Proteinstoffwechsel beeinflussende Hormone in physiologisch wirksamer Menge und/oder Fette oder Fettsäuren in einer Konzentration von 0 bis 300 g/l und/oder Peptide in einer Konzentration von 0 bis 100 g/l enthält.

## Claims

1. Aqueous dialysing and rinsing solution for intraperitoneal administration, containing electrolytes and additional osmotically active substances, optionally other additives, in which the total osmotic pressure is in the range of 300 to 700 mosm/l, characterised in that a mixture of the following components based on alpha-ketoacids in a (total) concentration of 1 to 70 g/l, the components are present in the following relative amounts:

| alpha-Ketoglutarate | 0 to 45 | parts by weight |
| Pyruvate (alpha-ketopropionate) | 0 to 45 | parts by weight |
| alpha-Ketosuccinate | 0 to 45 | parts by weight |
| 3-Methyl-alpha-ketovalerate | 6 to 9 | parts by weight |
| 4-Methyl-alpha-ketovalerate | 9 to 13.5 | parts by weight |
| 3-Phenylpyruvate | 0 to 9.5 | parts by weight |
| 3-Methyl-alpha-ketobutyrate | 13.5 to 20.5 | parts by weight |
| alpha-Ketoadipate | 0 to 10 | parts by weight |
| alpha-Ketobutyrate | 0 to 10 | parts by weight |

and in which the ratio of the alpha ketoacids 3-methyl-alpha-ketobutyrate : 4-methyl-alpha-ketovalerate : 3-methyl-alpha-ketovalerate is preferably 2.25:1.5:1.

2. A solution according to Claim 1, characterised in that the alpha-ketocarboxylic acids are used as the osmotically active substance as the calcium, potassium, magnesium or sodium salts, preferably the sodium salts, of these keto-carboxylic acids.

3. A solution according to Claim 1 or 2, characterised in that the osmotic pressure is preferably in the range of 320 to 550 mosm/l.

4. A solution according to Claims 1 to 3, characterised in that it contains the alpha-ketocarboxylic acids in an amount of 5 to 50 g/l, preferably 7.5 to 20 g/l.

5. A solution according to Claims 1 to 4, characterised in that the pH is 5.0 to 8.4.

6. A solution according to on of the Claims 1 to 5, characterised in that it contains the electrolytes in a concentration of 125 to 152 mmol/l $Na^+$, 0 to 8 mmol/l $K^+$, 0 to 3 mmol/l $Ca^{++}$, 0 to 2.5 mmol/l $Mg^{++}$, 10 to 80 mmol/l of ions chosen from the group lactate, acetate, bicarbonate and the corresponding remainder as chloride.

7. A solution according to one of the Claims 1 to 6, characterised in that it contains in addition a carboxylic acid salt chosen from the group succinate, fumarate, maleate, citrate or isocitrate, at a concentration of 0.5 to 20 g/l.

8. A solution according to one of the Claims 1 to 7, characterised in that it contains in addition the salt of an alpha-hydroxycarboxylic acid, preferably chosen from the group hydroxyphenylpyruvate, especially DL-2-hydroxy-4-methyl-thiobutyrate.

9. A solution according to one of the Claims 1 to 8, characterised in that it contains in addition 0.2 to 10 wt.% amino acids.

10. A solution according to one of the Claims 1 to 9, characterised in that it contains in addition 0.2 to 10 wt.% water soluble carbohydrates as additional osmotically active substances, preferably glucose, fructose, galactose, hydroxyethyl starch, dextran or glucose polymers.

11. A solution according to one of the Claims 1 to 10, characterised in that it contains in addition 10 to 500 mg/l vitamins, physiologically active amounts of hormones which influence the protein metabolism and/or fats or fatty acids at a concentration of 0 to 300 g/l and/or peptides at a concentration of 0 to 100 g/l.

**Revendications**

1. Solution aqueuse de dialyse et de rinçage pour administration intrapéritonéale, contenant des électrolytes et d'autres substances à action osmotique et éventuellement d'autres additifs, la pression osmotique totale se situant dans la gamme de 300 à 700 mosm/l,
solution caractérisée en ce que, dans un mélange des constituants suivants formés par des acides alpha-cétoniques (présents) en une concentration (totale) de 1 à 70 g/l, les constituants suivants sont présents en quantités relatives suivantes :

```
alpha-cétoglutarate                  0 à 45         parties en poids
pyruvate (alpha-cétopropionate)      0 à 45         parties en poids
alpha-cétosuccinate                  0 à 45         parties en poids
3-méthyl-alpha-cétovalérianate       6 à  9         parties en poids
4-méthyl-alpha-cétovalérianate       9 à 13,5       parties en poids
3-phénylpyruvate                     0 à 9,5        parties en poids
3-méthyl-alpha-cétobutyrate          13,5 à 20,5 parties en poids
alpha-cétoadipate                    0 à 10         parties en poids
alpha-cétobutyrate                   0 à 10         parties en poids
```

et le rapport entre les acides alpha-cétoniques :
le 3-méthyl-alpha-cétobutyrate: le 4-méthyl-alpha- cétovalérianate: le 3-méthyl-alpha-cétovalérianate vaut avantageusement 2,25:1,5:1.

2. Solution selon la revendication 1, caractérisée en ce que les acides alpha-cétocarboxyliques comme substances à action osmotique sont utilisés sous forme des sels de calcium, de potassium, de magnésium ou de sodium, avantageusement des sels de sodium, de ces acides cétocarboxyliques.

3. Solution selon la revendication 1 ou 2, caractérisée en ce que la pression osmotique se situe avantageusement dans la région de 320 à 550 mosm/l.

4. Solution selon une des revendications 1 à 3, caractérisée en ce que cette solution contient les acides alpha-cétocarboxyliques (présents) en une quantité de 5 à 50 g/l, avantageusement 7,5 à 20 g/l.

5. Solution selon l'une des revendications 1 à 4, caractérisée en ce que la valeur du pH vaut de 5,0 à 8,4.

6. Solution selon l'une des revendications 1 à 5, caractérisée en ce qu'elle contient des électrolytes en une concentration de 125 à 152 mmoles/l de $Na^+$, 0 à 8 mmoles/l de $K^+$, 0 à 3 mmoles/l de $Ca^{++}$, 0 à 2,5 mmoles/l de $Mg^{++}$, 10 à 80 mmoles/l d'ions choisis dans le groupe formé par du lactate, de l'acétate, du bicarbonate et le reste correspondant formé par du chlorure.

7. Solution selon l'une des revendications 1 à 6, caractérisée en ce qu'elle contient en outre un sel d'acide carboxylique choisi dans l'ensemble formé par un succinate, un fumarate, un malate, un citrate ou un isocitrate, en une concentration de 0,5 à 20 g/l.

8. Solution selon l'une des revendications 1 à 7, caractérisée en ce qu'elle contient en outre un sel d'acide alpha-hydroxycarboxylique, choisi avantageusement dans le groupe formé par de l'hydroxyphényl pyruvate, en particulier du DL-2-hydroxy-4-méthyl-thiobutyrate.

9. Solution selon l'une des revendications 1 à 8, caractérisée en ce qu'elle contient en outre 0,2 à 10 % en poids d'amino-acide(s).

10. Solution selon l'une des revendications 1 à 9, caractérisée en ce qu'elle contient en outre 0,2 à 10 % en poids de glucides, hydrosolubles comme autres substances à action osmotique, avantageusement du glucose, du fructose, du galactose, de l'hydroxyéthylamidon, des dextranes ou des polymères du glucose.

11. Solution selon l'une des revendications 1 à 10, caractérisée en ce qu'elle contient en outre 10 à 500 mg/l de vitamines, des hormones influençant le métabolisme des protéines et qui sont présentes en une quantité physiologiquement active/ou des graisses ou acides gras en une concentration de 0 à 300 g/l et/ou des peptides en une concentration de 0 à 100 g/l.